Europäisches Patentamt

European Patent Office

Office européen des brevets

(11) Publication number: **0 318 295 B1**

# EUROPEAN PATENT SPECIFICATION

(45) Date of publication of patent specification: **20.05.92**  (51) Int. Cl.⁵: **C07C 255/08**, C07C 253/24, B01J 27/057

(21) Application number: **88311148.6**

(22) Date of filing: **24.11.88**

(54) **Process for producing nitriles.**

(30) Priority: **25.11.87 JP 295054/87**
**18.02.88 JP 33999/88**
**09.08.88 JP 197126/88**

(43) Date of publication of application:
**31.05.89 Bulletin 89/22**

(45) Publication of the grant of the patent:
**20.05.92 Bulletin 92/21**

(84) Designated Contracting States:
**DE FR GB IT NL**

(56) References cited:
**FR-A- 2 173 203**
**GB-A- 1 334 859**
**GB-A- 2 090 156**
**US-A- 3 833 638**

(73) Proprietor: **MITSUBISHI KASEI CORPORATION**
**5-2, Marunouchi 2-chome Chiyoda-ku**
**Tokyo 100(JP)**

(72) Inventor: **Hatano, Masakatsu**
**2031-23 Kita-Hassaku-cho Midori-ku**
**Yokohama-shi Kanagawa-ken(JP)**
Inventor: **Kayo, Atsushi**
**5-1, Tsutsujigaoka Midori-ku**
**Yokohama-shi Kanagawa-ken(JP)**

(74) Representative: **Woods, Geoffrey Corlett et al**
**J.A. KEMP & CO. 14 South Square Gray's Inn**
**London WC1R 5EU(GB)**

## Description

The present invention relates to a process for producing nitriles. More particularly, it relates to an improved method for producing nitriles using an alkane as starting material.

Nitriles such as acrylonitrile and methacrylonitrile are industrially produced as important intermediates for the preparation of, for example, fibers, synthetic resins and synthetic rubbers. The most popular method for producing such nitriles is to subject an olefin such as propylene or isobutene to a reaction with ammonia and oxygen in the presence of a catalyst in the gaseous phase at a high temperature.

More recently, there have been proposed methods for producing acrylonitrile or methacrylonitrile using the so-called ammoxidation process, according to which a lower alkane such as propane or isobutane is subjected to a reaction with ammonia and oxygen in the presence of a catalyst in the gaseous phase. For instance, a method using a Mo type catalyst (JP-A-48-16887 (1973), JP-A-47-13312 (1972) (corresponding to GB-A-1,333,639) and JP-A-47-13313 (1972) (corresponding to US-A-3,833,638) and JP-B-55-42071 (1980)), a method using a V type catalyst JP-A-47-33783 (1972) and JP-A-52-148022 (1977), JP-B-50-23016 (1975) (corresponding to GB-A-1,336,135 and GB-A-1,336,136) and JP-B-47-51331 (1972) (corresponding to US-A-3,433,823)), a method using a Sb type catalyst (JP-B-45-4733 (1970) (corresponding to GB-A-1,194,855), JP-B-47-14371 (1972) (corresponding to US-A-3,670,008, US-A-3,678,090 and US-A-3,816,506), JP-B-50-17046 (1975) (corresponding to US-A-3,670,006, US-A-3,686,267 and US-A-3,746,737), JP-B-50-28940 (1975) (corresponding to GB-A-1,334,859), JP-B-56-47901 (1981), and US-A-3,686,295), and a method using other types of catalyst (JP-B-50-16775 (1975) (corresponding to US-A-3,652,638)) are known, but none of these methods is satisfactory regarding the selectivity of the objective nitriles.

In order to improve the selectivity of the nitriles, a small quantity of an organic halide, inorganic halide or sulfur compound has been added to the reaction system or water has been added to the reaction system. However, the former method has a problem of possible corrosion of the reaction apparatus while the latter method has a problem of formation of by-products by a side reaction.

Furthermore, the methods using conventional catalysts require a very high reaction temperature, which is about 500°C or higher, so that these methods are disadvantageous in terms of the reactor material which must be used and production cost.

The present inventors have made extensively researched methods of producing nitriles using an alkane as a starting material and, as a result, have found that by using a specific complex catalyst it was possible to produce the objective nitriles with a higher selectivity than attainable with conventional methods, with no need to introduce a halide or water into the reaction system and at a lower temperature (about 380 to 480°C) than that required in conventional methods.

The present invention provides a process for producing a nitrile which comprises subjecting an alkane and ammonia in the gaseous state to catalytic oxidation in the presence of a solid oxide catalyst comprising molybdenum, vanadium, tellurium and niobium.

The feature of the present invention lies in using a solid oxide catalyst comprising molybdenum (Mo), vanadium (V), tellurium (Te) and niobium (Nb) as essential components in the ammoxidation of an alkane. Typical examples of the catalyst are those of the empirical formula:

$$Mo_{1.0}V_aTe_bNb_cO_x$$

wherein a is from 0.01 to 1.0, b is from 0.01 to 0.5, c is from 0.01 to 1.0 and x represents a number such that the total valency of the metal elements is satisfied.

These catalysts can, for example, be prepared in the following way. Into an aqueous solution containing a given amount of ammonium metavanadate are added successively an aqueous solution of ammonium niobium oxalate, an aqueous solution of telluric acid and an aqueous solution of ammonium paramolybdate in such amounts that the atomic ratios of the respective metal elements fall in the ranges specified above, the mixture is heated and concentrated at about 70°C for about 30 minutes and then evaporated to dryness at 130°C. The resulting dry solid is calcined at a temperature of from 350 to 650°C, preferably from 350 to 450°C, for about 3 hours to give the desired catalyst.

In the above preparation process, the order of addition of the respective metal elements - molybdenum, vanadium, tellurium and niobium - is not specified, but it is desirable to add the molybdenum component, for example as an aqueous solution of ammonium paramolybdate, last of all as it faciliates obtaining a uniform aqueous solution.

EP 0 318 295 B1

In the above preparation, ammonium metavanadate may, for example, be replaced by $V_2O_5$, $V_2O_3$, $VOCl_3$ or $VCl_4$. Ammonium niobium oxalate may, for example be replaced by $NbCl_3$, $NbCl_5$ or $Nb_2(C_2O_4)_5$. Telluric acid may, for example, be replaced by $TeO_2$ and ammonium paramolybdate may, for example, be replaced by $MoO_3$, $MoCl_5$, phosphomolybdic acid or silicomolybdic acid. It is also possible to use a heteropolyacid which contains mixed-coodinate molybdenum and vanadium, such as molybdovanadophosphoric acid.

The contents of the metal elements constituting the catalyst used in the present invention are selected such that the atomic ratio of vanadium to molybdenum is from 0.01:1 to 1.0:1, preferably from 0.2:1 to 0.4:1, the atomic ratio of tellurium to molybdenum is from 0.01:1 to 0.5:1, preferably from 0.2:1 to 0.4:1 and the atomic ratio of niobium to molybdenum is from 0.01:1 to 1.0:1, preferably from 0.1:1 to 0.2:1.

Such a catalyst may be used either singly or in combination with a known carrier such as silica, alumina or aluminosilicate. The catalyst is worked into a suitable particle diameter and shaped according to the scale and system of the reaction and/or other factors in a manner commonly practiced in the art.

The alkane starting material is not particularly limited, for instance alkanes having 1 to 7 carbon atoms such as methane, ethane, propane, butane, isobutane, pentane, hexane or heptane may be mentioned. However, in consideration of the industrial use of the nitrile to be produced, it is preferred to use an alkane having 1 to 4 carbon atoms. The oxidation reaction in the process of the present invention is carried out by oxygen atoms existing in the catalyst or by the molecular oxygen supplied with the starting material gas.

In the case where molecular oxygen is supplied with the starting material, although pure oxygen gas may be used, since the purity of oxygen gas is not required, it is economical to use a molecular oxygen-containing gas such as air. In the case where molecular oxygen is not contained in the supplied gas as the starting material, it is preferable to supply alternately a gaseous mixture of alkane and ammonia and a molecular oxygen-containing gas to prevent the reductive deterioration of the catalyst, or to transfer used catalysts continuously into an ordinary oxidative regenerator to use the thus regenerated catalyst while using reactor of moving bed type.

As the reactor used in the process according to the present invention, any one of the reactors hitherto used in a gas phase contact catalytic reaction may be used. The introduction and extraction of the catalyst may be carried out as in a conventional process. The catalyst is usually used in an amount of from 0.02 to 2.4 $cm^3$, preferably from 0.1 to 0.5 $cm^3$ to one mole per hour of the supplied alkane.

The alkane, ammonia, the optional molecular oxygen-containing gas and a diluent gas optionally used for regulating the space velocity and the partial pressure of the oxygen may be supplied individually to the reactor. However, it is preferred to mix these substances in advance and to supply the thus prepared gaseous mixture to the reactor.

The amount of ammonia used in the reaction is generally from 0.5 to 3 mol, preferably from 0.8 to 1.5 mol, per one mol of alkane.

The amount of the molecular oxygen-containing gas which is used in the case of necessity is important concerning the selectivity of the nitriles. The molecular oxygen-containing gas is used so that the amount of molecular oxygen is generally not more than 5 mol, preferably from 1 to 3 mol, more preferably from 1 to 1.6 mol, per one mol of alkane.

As the diluent gas, an inactive gas such as nitrogen, argon or helium may be used. By increasing and decreasing the amount of the diluent gas used in the above-mentioned range, it is possible to regulate the space velocity and the partial pressure of oxygen in the supplied gas to a suitable range.

The space velocity of the supplied gas (a mixture of alkane, ammonia, the molecular oxygen-containing gas optionally used and the diluent gas optionally used) is generally from 100 to 10,000 $hr^{-1}$, preferably from 500 to 2,000 $hr^{-1}$.

In the present invention, the reaction of the alkane and ammonia is generally carried out at a temperature which is lower than the temperature of a conventional ammoxidation, namely at from 380 to 480°C, preferably from 400 to 450°C, under atmospheric pressure, a slightly increased pressure or a slightly reduced pressure.

In the present invention an $\alpha,\beta$-unsaturated nitrile such as methacrylonitrile or acrylonitrile is formed from isobutane and propane, acetonitrile is formed from ethane and hydrogen cyanide is formed from methane. In addition to these compounds, carbon monoxide, carbon dioxide and nitriles other than the objective nitrile are by-produced. However, the amount of production of the by-products is remarkably small.

The separation of the objective nitrile from the reaction mixture,and the purification of the thus separated nitrile can be carried out according to a conventional method.

The present invention is now further described in the following Examples.

3

The conversion (%) of an alkane and the selectivity (%) of a nitrile in the Examples and Comparative Examples are calculated by the following formulae:

$$\text{Conversion of alkane(\%)} = \frac{\text{mols of consumed alkane}}{\text{mols of supplied alkane}} \times 100$$

$$\text{Selectivity of objective nitrile(\%)} = \frac{\text{mols of objective nitrile obtained}}{\text{mols of consumed alkane}} \times 100$$

REFERENCE EXAMPLE 1 (Preparation of the catalyst):

Into 100 ml of warm water, 1170 mg of ammonium metavanadate were dissolved, and into the thus formed solution, 12.5 ml of an aqueous solution of ammonium niobium oxalate (0.2 Nb atom/litre), 10.0 ml of an aqueous solution of telluric acid (0.5 Te atom/litre) and 25.0 ml of an aqueous solution of ammonium paramolybdate (1.0 Mo atom/liter) were added to prepare a uniform aqueous solution.

The thus prepared aqueous solution was heated and then evaporated to dryness in a drier at 130°C to obtain a solid material.

The thus obtained solid material was calcined at 350° under a flow of air, and after molding the calcined material into a tablet 5 mm in diameter and 3 mm in length using a tablet machine, the tablet was pulverized and sifted into a powder of 16 to 28 mesh. The empirical formula of the thus prepared catalyst was:

$Mo_{1.0}V_{0.4}Te_{0.2}Nb_{0.1}O_{4.65}$

REFERENCE EXAMPLE 2 (Preparation of the catalyst):

In the same manner as in Reference Example 1 except for changing the amount of telluric acid, the following two catalysts were obtained:

$Mo_{1.0}V_{0.4}Te_{0.3}Nb_{0.1}O_{4.85}$

and

$Mo_{1.0}V_{0.4}Te_{0.4}Nb_{0.1}O_{5.05}$

EXAMPLES 1 to 4:

After charging a reactor with 0.5 cm$^3$ of the catalyst obtained in Reference Example 1, a gaseous mixture of propane, ammonia, air and nitrogen in a molar ratio shown in Table 1 was supplied into the reactor at a space velocity of 1400 hr$^{-1}$ to carry out a gas phase catalytic reaction at 422°C. The results are shown in Table 1.

4

TABLE 1

| EXAMPLE | Composition of gas (molar ratio) | | | | Conversion of propane (%) | Selectivity of acrylonitrile (%) |
|---|---|---|---|---|---|---|
| | Propane | NH$_3$ | Air | N$_2$ | | |
| 1 | 1 | 1.2 | 12.4 | 2.4 | 24.4 | 51.8 |
| 2 | 1 | 1.2 | 10.4 | 4.9 | 22.6 | 61.5 |
| 3 | 1 | 1.2 | 7.6 | 7.3 | 20.9 | 71.6 |
| 4 | 1 | 1.2 | 5.1 | 9.8 | 11.0 | 78.4 |

COMPARATIVE EXAMPLE 1:

While using a catalyst of the formula shown in Table 2 prepared in the same manner as in Reference Example 1 except for not using the Nb component, a gaseous mixture of propane, ammonia, air and nitrogen of the same composition as in Example 1 was supplied into a reactor at the same space velocity as in Example 1 to carry out the reaction at the temperature shown in Table 2. The results are shown in Table 2.

COMPARATIVE EXAMPLE 2:

While using a catalyst of the formula shown in Table 2 prepared in the same manner as in Reference Example 1 except for not using the Te component, a gaseous mixture of propane, ammonia, air and nitrogen of the same composition as in Example 1 was supplied into a reactor at the same space velocity as in Example 1 to carry out the reaction at the temperature shown in Table 2. The results are shown in Table 2.

COMPARATIVE EXAMPLE 3:

While using a catalyst of the formula shown in Table 2 prepared in the same manner as in Reference Example 1 except for not using the V component, a gaseous mixture of propane, ammonia, air and nitrogen of the same composition as in Example 1 was supplied into the reactor at the same space velocity as in Example 1 to carry out the reaction at the temperature shown in Table 2. The results are shown in Table 2.

COMPARATIVE EXAMPLE 4:

While using a catalyst of the formula shown in Table 2 prepared in the same manner as in Reference Example 1 except for not using the Mo component, a gaseous mixture of propane, ammonia, air and nitrogen of the same composition as in Example 1 was supplied into a reactor at the same space velocity as in Example 1 to carry out the reaction at the temperature shown in Table 2. The results are shown in Table 2.

From a comparison of Examples 1 to 4 and Comparative Examples 1 to 4, it can be understood that Mo, V, Te and Nb are all indispensable components in the catalyst for obtaining high selectivity.

TABLE 2

| Comparative Example | Catalyst (atomic ratio) | Temperature (°C) | Conversion of propane (%) | Selectivity of acrylonitrile (%) |
|---|---|---|---|---|
| 1 | Mo$_{1.0}$V$_{0.4}$Te$_{0.2}$O$_{4.4}$ | 464 | 7.6 | 2.0 |
| 2 | Mo$_{1.0}$V$_{0.4}$Nb$_{0.1}$O$_{4.25}$ | 417 | 34.2 | 10.5 |
| 3 | Mo$_{1.0}$Nb$_{0.1}$Te$_{0.2}$O$_{3.65}$ | 423 | 0.3 | trace |
| 4 | V$_{0.4}$Nb$_{0.1}$Te$_{0.2}$O$_{1.65}$ | 421 | 2.4 | trace |

EXAMPLE 5:

After charging a reactor with 1 cm$^3$ of the catalyst obtained in Reference Example 1, a gaseous mixture of propane, ammonia and nitrogen in the molar ratio of 1:1.2:14.9 was supplied to the reactor at the space velocity of 700 hr-1 for 5 minutes to carry out the gas phase contact catalytic reaction at 401°C.

The conversion was 9.9 % and the selectivity was 76.3 %.

Although in this Example the oxidation reaction of propane was carried out without supplying molecular oxygen, acrylonitrile was obtained at a high selectivity only through an oxidation reaction using the oxygen atoms which existed in the catalyst.

EXAMPLES 6 and 7:

While using 0.5 cm$^3$ of each of the two catalysts obtained in Reference Example 2 respectively and supplying a gaseous mixture of propane, ammonia, air and nitrogen in the molar ratio of 1:1.2:7.6:7.3 at the space velocity of 1400 hr$^{-1}$ to a reactor, the gas phase contact catalytic reaction was carried out at 422°C.

The results are shown in Table 3.

TABLE 3

| Example | Catalyst (atomic ratio) | Temperature (°C) | Conversion of propane (%) | Selectivity of acrylonitrile (%) |
|---|---|---|---|---|
| 6 | $Mo_{1.0}V_{0.4}Te_{0.3}Nb_{0.1}O_{4.85}$ | 423 | 20.2 | 60.9 |
| 7 | $Mo_{1.0}V_{0.4}Te_{0.4}Nb_{0.1}O_{5.05}$ | 424 | 15.2 | 52.7 |

EXAMPLES 8 to 10:

After charging a reactor with 0.5 cm$^3$ of the catalyst obtained in Reference Example 1, a gaseous mixture of isobutane, ammonia, air and nitrogen in a composition shown in Table 4 was supplied to the reactor at a space velocity of 1400 hr$^{-1}$ to carry out the gas phase contact catalytic reaction at 448°C. The results are shown in Table 4.

TABLE 4

| EXAMPLE | Composition of gas (molar ratio) | | | | Conversion of isobutane (%) | Selectivity of methacrylonitrile (%) |
|---|---|---|---|---|---|---|
| | Isobutane | NH$_3$ | Air | N$_2$ | | |
| 8 | 1 | 1.2 | 7.6 | 7.3 | 15.2 | 22.4 |
| 9 | 1 | 1.2 | 5.1 | 9.8 | 11.0 | 42.7 |
| 10 | 1 | 1.2 | 2.7 | 12.2 | 6.0 | 41.6 |

**Claims**

1. A process for producing a nitrile which comprises subjecting an alkane and ammonia in the gaseous state to catalytic oxidation in the presence of a solid oxide catalyst comprising molybdenum, vanadium, tellurium and niobium.

2. A process according to claim 1 wherein the catalyst has the empirical formula:

$Mo_{1.0}V_aTe_bNb_cO_x$

6

wherein a is from 0.01 to 1.0, b is from 0.01 to 0.5, c is from 0.01 to 1.0 and x represents a number such that the total valency of the metal elements is satisfied.

3.   A process according to claim 2 wherein a is from 0.2 to 0.4, b is from 0.2 to 0.4 and c is from 0.1 to 0.2.

4.   A process according to any one of the previous claims which is carried out in the presence of molecular oxygen.

5.   A process according to any one of the preceding claims wherein the alkane contains from 1 to 4 carbon atoms.

6.   A process according to any one of the preceding claims wherein the catalyst is present in an amount of from 0.02 to 2.4 $cm^3$ per 1 mole per hour of the alkane.

7.   A process according to any one of the preceding claims wherein the ammonia is present in an amount of from 0.5 to 3 mol per 1 mol of alkane.

8.   A process according to any one of the preceding claims which is carried out at a temperature of from 380 to 480°C.

## Revendications

1.   Procédé de production d'un nitrile qui comporte l'exposition d'un alcane et d'ammoniaque à l'état gazeux à une oxydation catalytique en présence d'un catalyseur oxydé à l'état solide comprenant du molybdène, du vanadium, du tellure et du niobium.

2.   Procédé selon la revendication 1, dans lequel la formule empirique du catalyseur est la suivante:

$$Mo_{1,0}V_aTe_bNb_cO_x$$

dans laquelle a est de 0,01 à 1,0, b est de 0,01 à 0,5, c de 0,01 à 1 et x représente un nombre tel que la totalité des valences des éléments métalliques soit satisfaites..

3.   Procédé selon la revendication 2 dans lequel a est de 0,2 à 0,4, b est de 0,2 à 0,4 et c est de 0,1 à 0,2.

4.   Procédé selon l'une des revendications précédentes effectué en présence d'oxygène moléculaire.

5.   Procédé selon l'une des revendications précédentes dans lequel l'alcane contient de 1 à 4 atomes de carbone.

6.   Procédé selon l'une des revendications précédentes dans lequel le catalyseur est présent en quantité de 0,02 à 2,4 $cm^3$ pour une mole d'alcane par heure.

7.   Procédé selon l'une des revendications précédentes dans lequel l'ammoniaque est présent dans une quantité de 0,5 à 3 moles par mole d'alcane.

8.   Procédé selon l'une des revendications précédentes effectué à une température de 380 à 480°C.

## Patentansprüche

1.   Verfahren zur Herstellung eines Nitrils, umfassend die Durchführung einer katalytischen Oxidation mit einem Alkan und Ammoniak in dem gasförmigen Zustand in der Gegenwart eines festen Oxidkatalysators, der Molybdän, Vanadium, Tellur und Niob umfasst.

**2.** Verfahren nach Anspruch 1, dadurch **gekennzeichnet,** dass der Katalysator die empirische Formel aufweist:

$$Mo_{1.0}V_aTe_bNb_cO_x$$

worin a 0,01 bis 1,0 ist, b 0,01 bis 0,5 ist, c 0,01 bis 1,0 ist und x eine Zahl darstellt, so dass die Gesamtvalenz der Elemente erfüllt ist.

**3.** Verfahren nach Anspruch 2, dadurch **gekennzeichnet,** dass a 0,2 bis 0,4 ist, dass b 0,2 bis 0,4 ist und dass c 0,1 bis 0,2 ist.

**4.** Verfahren nach einem der vorhergehenden Ansprüche, dadurch **gekennzeichnet,** dass es in der Gegenwart von molekularem Sauerstoff durchgeführt wird.

**5.** Verfahren nach einem der vorhergehenden Ansprüche, dadurch **gekennzeichnet,** dass das Alkan 1 bis 4 Kohlenstoffatome enthält.

**6.** Verfahren nach einem der vorhergehenden Ansprüche, dadurch **gekennzeichnet,** dass der Katalysator in einer Menge von 0,02 bis 2,4 $cm^3$ pro 1 Mol pro Stunde des Alkans vorhanden ist.

**7.** Verfahren nach einem der vorhergehenden Ansprüche, dadurch **gekennzeichnet,** dass der Ammoniak in einer Menge von 0,5 bis 3 Mol/1 Mol Alkan vorhanden ist.

**8.** Verfahren nach einem der vorhergehenden Ansprüche, dadurch **gekennzeichnet,** dass es bei einer Temperatur von 380 bis 480 °C durchgeführt wird.